# EUROPEAN PATENT SPECIFICATION

(11) **EP 4 528 218 B1**
(45) Date of publication and mention of the grant of the patent: **04.03.2026**
(21) Application number: 23199141.5
(22) Date of filing: 22.09.2023
(51) Int. Cl.: G01B 11/24, G06T 7/55, G01S 7/481, G01S 17/42, G01S 17/66, G01S 17/86, G01S 17/89, A61B 34/20, G01S 7/48

(54) **TRACKING MARKER, MOBILE DEVICE, POST PROCESSING UNIT AND METHOD FOR SCANNING A 3D SPACE**
VERFOLGUNGSMARKER, MOBILES GERÄT, NACHVERARBEITUNGSEINHEIT UND VERFAHREN ZUM SCANNEN EINES 3D-RAUM
MARQUEUR DE SUIVI, DISPOSITIF MOBILE, UNITÉ DE POST-TRAITEMENT ET PROCÉDÉ DE BALAYAGE D'UN ESPACE 3D

(43) Date of publication of application: 26.03.2025
(73) Proprietor: Deutsche Telekom AG, 53113 Bonn (DE)
(72) Inventor: YUN, Harrison Hongseo, 12203 Berlin (DE); ARUNOV, Elmar, 10587 Berlin (DE)
(74) Representative: Vossius & Partner Patentanwälte Rechtsanwälte mbB

(56) References cited:
- US-A1- 2011 102 550
- US-A1- 2019 066 338
- US-A1- 2022 215 934

## Description

The present disclosure relates to a tracking marker and a method for use thereof in a system, and a method for scanning a three dimensional space. It also relates to a mobile device and a post processing unit for use with a mobile device.

In detail, the present disclosure relates to a method and system for three dimensional, 3D, space scanning and modeling based on a tracking marker integrated with a color checker, also known as a color correction card.

3D space scanning and modeling is a technique that uses advanced technology to capture and create a 3D digital model of a physical space.

This technology can be used in various industries such as architecture, engineering, and entertainment to create realistic visualizations and simulations. The photorealistic 3D space scanning market is expected to grow significantly in the coming years. A report says that the market size for 3D scanning is expected to reach USD 5.9 billion by 2025, growing at a Compound Annual Growth Rate of 7.8% from 2020 to 2025.

This growth can be attributed to the increasing demand for 3D scanning technology in various industries such as automotive, aerospace, and healthcare, among others.

The need for accurate and detailed 3D models for visualization, simulation, and analysis purposes is also driving growth of the market.

Tracking marker technology is a technique used to track the position of objects in a three-dimensional space. This technology involves placing a plurality of markers on the objects, which are then tracked by a camera. It has been already proven to be a useful tool in various industries such as film, gaming, and advertising.

With the advancements in computer vision and machine learning, tracking marker technology is becoming more accurate and efficient, allowing for a more realistic and immersive experience.

A color checker, also known as a color correction card, is a reference tool used to ensure that the colors in an image or video are accurate and consistent. It is typically a small card or chart that contains a set of color swatches, which are designed to represent a range of colors that are commonly found in real-world scenes.

The color checker is photographed or filmed alongside the scene or subject, and the resulting image or footage is then used as a reference for color correction during post-production.

By comparing the colors in the image or footage with the colors on the color checker, colorists can adjust the color balance and saturation to achieve a more accurate and consistent look.

The use of a color checker is particularly important in applications such as film and photography where color accuracy is critical.

However, in case that 3D space is scanned by a depth sensor and optical camera in a mobile phone, there are several technical problems reported.

3D space scanning requires capturing multiple images with different angles and distances in order to cover a wide range of space, i.e., from bottom, i.e., floor, to top, i.e., ceiling, and up to 360 degrees of horizontal direction.

In this case, color inconsistency is a common problem that can occur when capturing images in 360 degrees of view. This is because the lighting conditions and color temperature can vary significantly across the entire space, resulting in color variations that can be difficult to correct in post-production. In addition, the camera's white balance settings may not be able to accurately capture the full range of colors in the scenes, leading to color shifts and inconsistencies. Another factor that can contribute to color inconsistency is the texture mapping process used to combine the individual images into a seamless 360-degree of mesh object.

If the texture mapping is not done correctly, it can result in a visible seams and color mismatches between adjacent images.

To minimize color inconsistency when capturing images in 360 degrees of view, it is important to use a camera with a wide dynamic range and accurate white balance settings. It may also be necessary to use additional lighting equipment or adjust the camera's exposure settings to ensure consistent lighting conditions across the entire scene.

However, it was found that it is not possible to figure out the right but fixed settings to remove any inconsistency due to the nature of light movement indoor. Furthermore, color inconsistency is still found in the images which are captured with disabling all camera setting parameters such as exposure and shutter speed.

In general, careful attention can be paid to the texture mapping process, using software tools that can automatically correct for color and exposure variations between adjacent images. However, the structure and shape of 3D space with 360 degrees of view are a lot more complicated so it is harder than doing a color correction for a single image or panoramic images.

Position tracking or SLAM, Simultaneous Localization and Mapping, using a mobile phone alone can suffer from drift errors due to several factors that can affect the accuracy of the position tracking. One of the main factors is the limitations of the sensors built into the mobile phone, such as the accelerometer, gyroscope, and magnetometer.

These sensors are not as accurate as those found in dedicated tracking devices, which can result in errors in the position tracking over time. Another factor that can contribute to drift errors is the quality of the feature detection and matching algorithms used in the SLAM system.

These algorithms are used to identify and track key features in the environment, which can be used to estimate the position and orientation of the mobile phone. However, if these algorithms are not robust enough or are not properly calibrated, they may produce errors in the position tracking, leading to drift over time.

Finally, environmental factors such as changes in lighting conditions or the presence of reflective surfaces can also affect the accuracy of the position tracking, leading to drift errors.

Said poor position tracking and/or SLAM performance prevents from the mass and direct utilization of a mobile phone in 3D space scanning and modeling.

US 2011/102550 A1 relates to an apparatus and method for computing a three dimensional model of a surface of an object using.at least one localisation template having predetermined geometrical features.

US 2019/066338 A1 relates to a color detection system, comprising: a card having printed thereon a color chart comprising multiple colored areas, and at least one grayscale area; a camera configured to acquire an image comprising a pixel representation a sample positioned adjacent to the card; a storage device configured to store reference data corresponding to the color chart and the at least one grayscale area; and a processor configured to: perform a grayscale correction to the pixel representation of the color chart and sample using a grayscale correction transformation.

US 2022/215934 A1 relates to systems, methods, and devices for providing wound capturing guidance.

The invention is set out in the appended set of claims. Any references in the following description to embodiments, objects, aspects and/or examples which are not covered by the appended claims are considered as not being part of the present invention.

According to an aspect of the present disclosure, there is provided a tracking marker comprising: a fiducial section for providing a reference scale in at least two dimensions and an orientation reference; and at least one color swatch with a plurality of color swatch elements for providing a color reference, wherein the tracking marker is preferably configured to be attached to an object in a 3D space.

In an embodiment of the present disclosure the at least one color swatch comprises at least one of: a set of natural colors, a set of primary and secondary colors, a set of grayscale colors, and a set of miscellaneous colors, wherein preferably each color swatch comprises six or more colors swatch elements.

In an embodiment of the present disclosure the tracking marker is a two-dimensional tracking marker and the fiducial section provides a reference scale in each of the two dimensions of the tracking marker.

According to the invention the fiducial section is a cut-out in a central portion of the tracking marker, and the at least one color swatch is arranged to surround the fiducial section.

The tracking marker has a square shape with a side length A, and the tracking marker area is divided into n by m rectangular area elements with side lengths of A/n in a first direction and A/m in a second direction, with n and m being integer numbers, with n being preferably 6 and m being preferably 5.

The tracking marker comprises four color swatches and each color swatch comprises a same number of color swatch elements each corresponding to one rectangular area element, and the fiducial section is a cut-out of the same number of rectangular area elements in the central portion of the tracking marker.

In an embodiment of the present disclosure the rectangular area elements are arranged in m=6 columns in the first direction and n=5 rows in the second direction, wherein a first color swatch is arranged on the rectangular area elements of the first row, wherein a second color swatch is arranged on the rectangular area elements of the fifth row; wherein a third color swatch is arranged on the rectangular area elements of the second, third, and fourth row in the first and second column; wherein a fourth color swatch is arranged on the fourth to sixth rectangular area elements of the second row, on the sixth element of the third row, and the fifth and sixth rectangular area element of the fourth row, and wherein the remaining rectangular area elements are forming the cut-out for the fiducial section.

According to an aspect of the present disclosure, there is provided a mobile device for scanning a 3D space provided with a plurality of tracking markers according to any one of the preceding aspects and embodiments, the mobile device comprising: a camera configured to acquire a plurality of digital images of at least a part of the 3D space; and a depth sensor configured to acquire corresponding depth data of the 3D space, wherein the mobile device comprises or is connected to a post processing unit configured to correct the colors and/or white balance of the acquired images based on the at least one color swatch of the tracking markers; and perform texture mapping based on the acquired images and based on position tracking data based on the fiducial section of the tracking markers.

According to an aspect of the present disclosure, there is provided a post processing unit for use with a mobile device according to claim 8 for scanning a 3D space provided with a plurality of tracking markers according to any one of the preceding aspects and embodiments, wherein the post processing unit is configured to correct the colors and/or white balance of the acquired images based on the at least one color swatch of the tracking markers; and to perform texture mapping based on the acquired images and based on position tracking data based on the fiducial section of the tracking markers.

According to an aspect of the present disclosure, there is provided a system for scanning a 3D space comprising: a plurality of tracking markers according to any one of the preceding aspects and embodiments arranged in the 3D space; a mobile device according to the above aspect; and a post processing unit according to the above aspect.

According to an aspect of the present disclosure, there is provided a method for scanning a 3D space provided with a plurality of tracking markers according to any one of the preceding aspects and embodiments, the method comprising: acquiring with a camera of a mobile device a plurality of images of at least a part of the 3D space; acquiring corresponding depth data of the 3D space with a depth sensor of the mobile device; and performing a post processing comprising correcting the colors and/or white balance of the acquired images based on the at least one color swatch of the tracking markers; and performing texture mapping based on the acquired images and based on position tracking data based on the fiducial section of the tracking markers.

It is a general concept of the present disclosure to provide a tracking marker for 3D scanning that combines the features of a tracking marker for position tracking and the features of a color checking card.

It is a further general concept that the 3D scanning result is improved based on color correction and more accurate position tracking in images acquired with a mobile phone.

Thus, the present disclosure enables the use of a mobile phone in a 3D scanning system and method.

Other aspects, features, and advantages will be apparent from the summary above, as well as from the description that follows, including the figures and the claims.

The system and method according to the present disclosure may enhance color and white balance calibration (correction), and also improves the position tracking of images captured from a mobile optical camera.

In an aspect of the invention the tracking marker is preferably configured to attached to an object in a 3D space. To this end the tracking marker be provided on a substrate configured to be attached to a surface of the object. Preferably, the substrate is a paper or plastic material on which the tracking marker according to the present invention is printable.

The attachment may be releasable or permanent. The attachment may be an adhesive connection. The attachment may be a velcro connection. The attachment may be an electrostatic connection. The attachment may be a magnetic connection.

The disclosure will be further described with reference to the figures. Therein,
- Fig. 1: shows an embodiment of the tracking marker according to an embodiment of the present disclosure in color;
- Fig. 2: shows the embodiment of the tracking marker of Fig. 1 with names and hex color codes of the colors;
- Fig. 3: shows the position of color swatches on a tracking marker according to an embodiment of the present disclosure;
- Fig. 4: shows the fiducial marker on a tracking marker according to an embodiment of the present disclosure;
- Fig. 5: shows an installation of tracking markers according to an embodiment of the present disclosure in a 3D space for 3D scanning according to an embodiment of the present disclosure;
- Fig. 6: shows an 3D scanning motion according to an embodiment of the present disclosure; and
- Fig. 7: shows a flow diagram of a 3D scanning method according to an embodiment of the present disclosure.

In an embodiment of the present disclosure, a tracking marker integrated with a color checker is provided. This tracking marker may be used in a system and/or method for 3D space scanning and modeling through the most efficient, simplest, and direct way.

Conventional color checkers are popular among professional photographers and designers, because they provide a reliable and consistent reference for color accuracy. By using a color checker during a photo shoot, photographers and designers can ensure that the colors in their images are accurate and consistent, which is critical for maintaining the integrity of the final product.

Color checkers are particularly useful in situations where lighting conditions can vary significantly, such as indoor shoots with mixed lighting sources. By including a color checker in a scene, photographers and designers can capture an accurate reference of the colors in the scene, which can be used to correct for any color shifts or inconsistencies during post-production.

Also, tracking markers have several advantages over other position tracking technologies, including:
Flexibility: Tracking markers can be placed on any object, making them flexible solution for a wide range of applications. This is particularly useful in situations where the objects being tracked are irregularly shaped or have complex movements.
Accuracy: Tracking markers can provide highly accurate position tracking, particularly when used in conjunction with high-quality cameras and tracking software. This makes them an ideal solution for applications that require precise movement tracking, such as motion capture for film and gaming.
Low latency: Tracking markers can provide real-time position tracking with low latency, making them suitable for applications that require fast response times, such as robotics or virtual reality.
Cost efficiency: Tracking markers are often a more cost-effective solution than other position tracking technologies, such as GPS or LiDAR. This makes them a popular choice for applications where cost is a consideration.
Easy to set up: Tracking markers are relatively easy to set up and use, requiring only a camera and the markers themselves. This makes them a convenient solution for applications that require quick and easy setup, such as temporal installations.

Therefore, in an aspect of the present disclosure a tracking marker is combined with a color checker so that it can resolve two major technical challenges of 3D scanning, color inconsistency and poor position tracking performance.

This is particularly relevant when non-specific hardware such as a mobile device, preferably a smartphone is used. A smartphone may have sufficient computing power, especially when connected to edge computing resources or cloud computing resources. However, the images of build-in cameras, when used in auto focus and/or auto setting mode, which is the only mode really usable via typical API's, frequently suffer from color inconsistency. Therefore, also the position tracking performance is reduced.

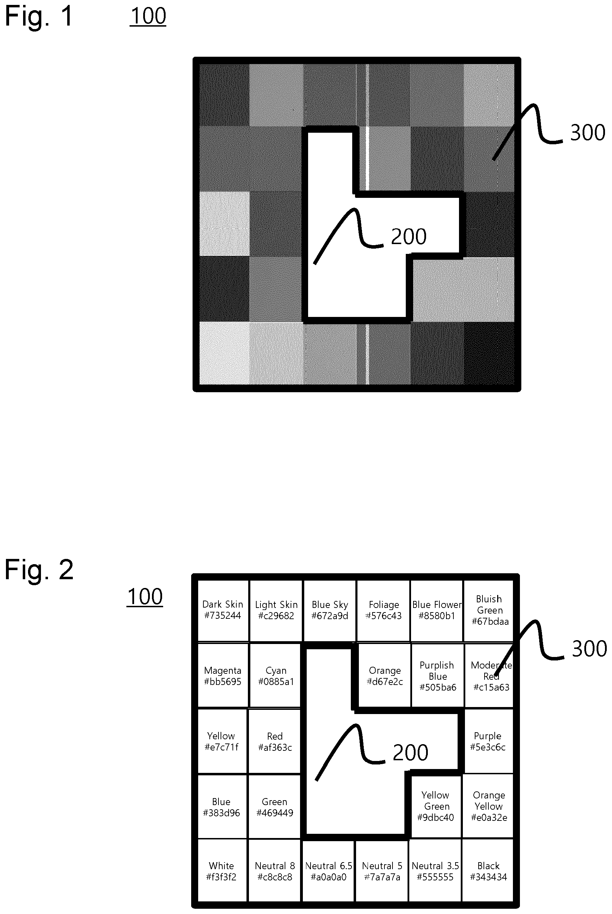

Fig. 1 shows an embodiment of the tracking marker according to an embodiment of the present disclosure in colors and Fig. 2 shows the embodiment of the tracking marker of Fig. 1 with names and hex color codes of the colors.

In detail, the tracking marker 100 comprises a solid area 300 and a cut-out 200. The solid area 300 comprises at least one color swatch with a plurality of color swatch elements.

It is noted that Fig.1 shows a color representation of the color swatches 300. Fig. 2 shows the same embodiment with a name of the respective color as well as a hex color code of the respective color.

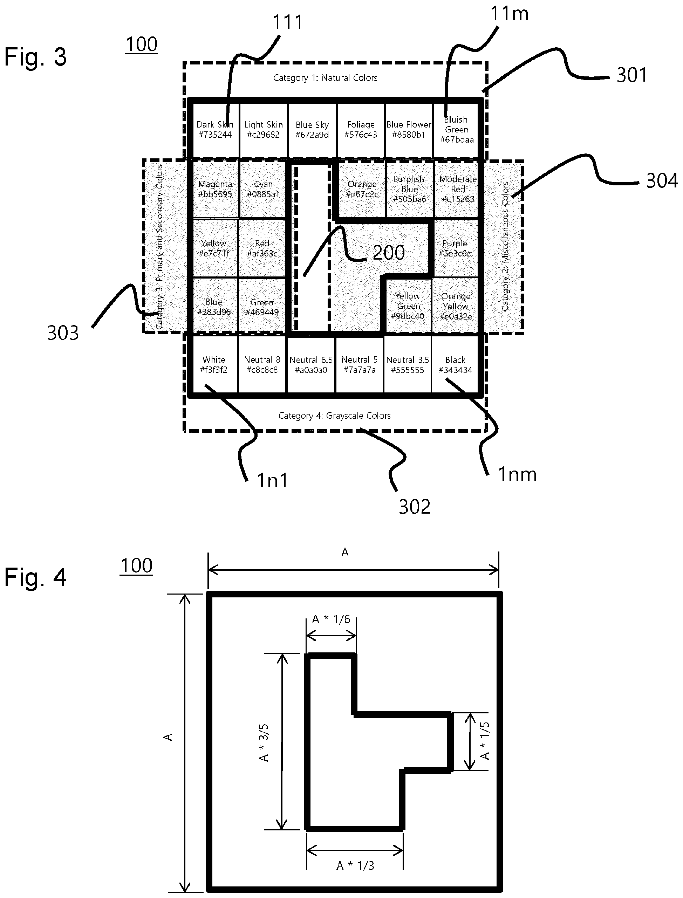

Fig. 3 shows the position of color swatches on a tracking marker according to an embodiment of the present disclosure.

The tracking marker comprises four color swatches 301, 302, 303 and 304 arranged in the solid area 300 of the tracking marker 100.

The first color swatch 301 comprises natural colors. The second color swatch 302 comprises grayscale colors. The third color swatch 303 comprises primary and secondary colors. The fourth color swatch 304 comprises miscellaneous colors.

Fig. 4 shows the fiducial marker on a tracking marker according to an embodiment of the present disclosure. A fiducial marker is formed by a cut-out 200 in the tracking marker 100. The cut-out is arranged in a central portion of the tracking marker 100. The fiducial marker is surrounded by the color swatches.

It is noted that the term cut-out encompasses a physical cut-out, i.e., removed material, as well as a relative cut-out. The latter being an area visually significantly different from the surrounding area, e.g., by a different color, preferably solid white, black, or green.

The tracking marker 100 has a square shape with a side length of A. The tracking marker can be divided into m columns of equal width and n rows of equal height. n and m being integer numbers.

The applicants have realized that the square shape and the discrete division into rectangular sub-elements improves the performance with typical image recognition algorithms. Thus, the color correction and position tracking performance are improved.

In other words, the tracking marker 100 may be divided into n by m rectangular surface area elements 111 to 1nm. Each rectangular surface area element corresponding to a color swatch element or a portion of the cut-out.

In a preferred embodiment of the present disclosure, n is 5 and m is 6, that is the rectangular area elements are arranged in m=6 columns in the first direction and n=5 rows in the second direction.

The number of rectangular area elements is the same in each of the color swatch and the cut-out.

In a preferred embodiment of the present disclosure, a first color swatch is arranged above the cut-out, a second color swatch is arranged below the cut-out, a third and fourth color swatch is arranged left and right of the cut-out. That is, the color swatches surround the fiducial marker.

In a preferred embodiment of the present disclosure, the first color swatch 301 is arranged on the rectangular area elements of the first row. The second color swatch 302 is preferably arranged on the rectangular area elements of the fifth row. The third color swatch 303 is preferably arranged on the rectangular area elements of the second, third, and fourth row in the first and second column. The fourth color swatch 304 is preferably arranged on the fourth to sixth rectangular area elements of the second row, on the sixth element of the third row, and the fifth and sixth rectangular area element of the fourth row. The remaining rectangular area elements are preferably forming the cut-out for the fiducial section 200.

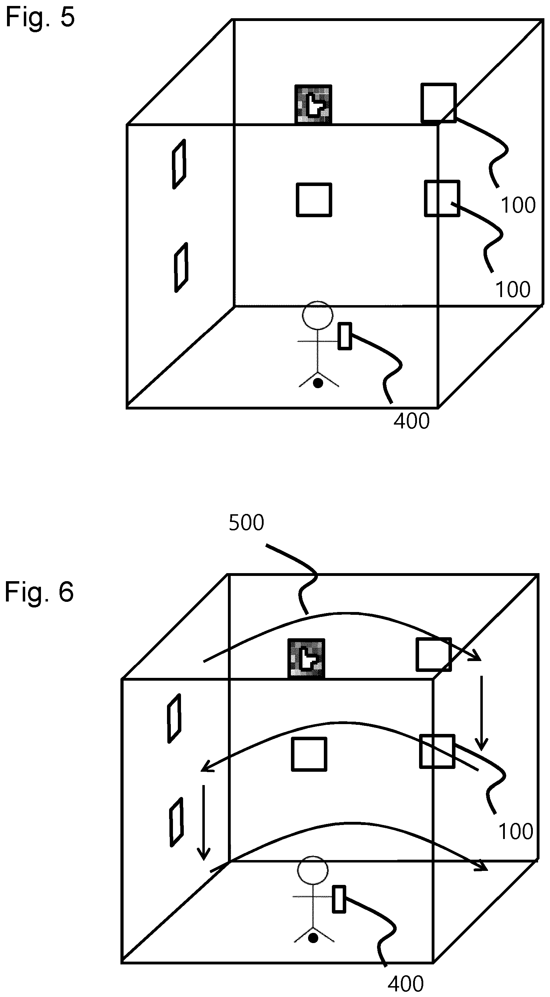

Fig. 5 illustrates an example that shows how to attach tracking markers integrated with a color checker in a 3D space, preferably an indoor space. Preferably, the markers are provided such that at least one marker is visible in each image of the 3D scanning as discussed above. That is, the markers are provided about equally spaced in every view direction of the 3D scan, preferably the complete 3D space.

Fig. 6 illustrates an example that shows how to scan a 3D space provided with tracking markers integrated with a color checker. In detail, the 3D space is scanned by a use of a hand-held mobile device 400, preferably a mobile phone. The mobile device is moved, as illustrated by the arrows 500, such that the build-in sensors, i.e. the depth sensor and/or camera captures a plurality of data and/or images covering the complete view direction of the 3D scan, preferably the complete 3D space.

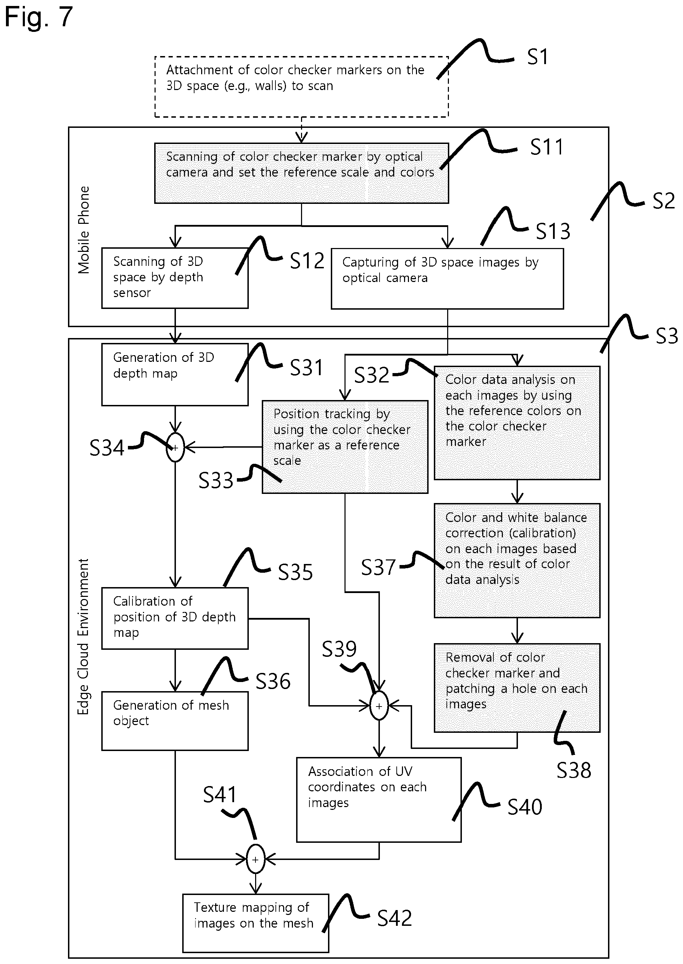

Fig. 7 shows a flow diagram of a 3D scanning method according to an embodiment of the present disclosure. In detail, the method according to an embodiment of the present disclosure comprises the steps of providing tracking markers according to the present disclosure in the 3D space S1; the step of scanning the 3D space S2; the step of processing the scanned data S3.

First the tracking markers, as discussed above with respect to Figs. 1 to 4, are provided S1 in the 3D space, as discussed with respect to Fig. 6, to be scanned.

Next the 3D scanning is performed S2. That is, optionally first in an initialization step S11 at least one tracking marker is imaged with the camera of the mobile device and the scale and color settings of the camera are set based on the fiducial marker and/or the at least one color swatch S11. Alternatively, the camera may be used in auto-setting mode.

Based on the above settings the scanning with the depth sensor S12 and the acquisition of a plurality of images of the 3D space S13 is performed. Furthermore, the acquisition of a plurality camara images S13 is performed. The acquisition may include capturing, pre-processing, combining and/or saving a plurality of images of the 3D space. The acquisition may be performed during a continuous sweeping motion or stepwise, with or without additional guiding information, in order to sufficiently cover the entire scan area.

Next the obtained depth data from step S12 and the obtained images from step S13 is post-processed to perform texture mapping and thus obtain 3D content from the 3D scan.

In step S31, a 3D depth map is generated based on the data from the depth sensor.

In step S32, a color data analysis is performed for each image of the plurality of images from step S13 based on the reference colors on the color swatches of the tracking markers.

In step S33, a position tracking is performed for each image of the plurality of images from step S13 based on the fiducial marker on the tracking markers.

In other words, the original data about colors and scale based on the fiducial marker shall be the reference or fiducial to analyze the color inconsistency and measure the distance and the pose of the object.

In step S34, the position tracking data based on the fiducial marker on the tracking markers and the depth map data of step S31 is combined to calibrate a corrected 3D depth map in step S35.

That is, after step S31, it is possible to have an initial 3D depth map. However, again, due to the poor performance of SLAM at a mobile device, e.g. an iPhone, the initial 3D depth map will have many errors.

Now, through step S33 process, the precise distance data is analyzed and the precision of the 3D depth map is improved. Between two distance data, one from SLAM and the other from the marker, the distance measured from the marker has a higher priority.

In step 36, a mesh object is generated based on the calibrated 3D depth map of step S35.

In step 37, the plurality of images is color and/or white balance corrected based on the results of the color data analysis of step S32.

In step 38, the tracking markers are removed from the plurality of images and the resulting holes a patched.

In step 39, the calibrated 3D depth map, the position tracking data and the corrected images are combined for the association of UV coordinated on each image in step S40.

As noted before, a UV texture mapping process requires higher precision data. If the data would be of poor quality, e.g. merely the SLAM data, texture fragmentation or texture tearing with many broken seam and other inconsistencies will be observed.

Finally the mesh object and UV coordinates are combined S41 and Texture mapping is performed in step S42.

It is noted that steps S2 and S3 may be performed on the mobile device 400. Alternatively, the post processing or parts thereof may be performed in connected processing instances, preferably in an edge cloud environment.

While various embodiments of the present disclosure have been described above, it should be understood that they have been presented by way of example only, and not by way of limitation. Likewise, the various diagrams may depict an example architectural or configuration, which are provided to enable persons of ordinary skill in the art to understand exemplary features and functions of the present disclosure. Such persons would understand, however, that the present disclosure is not restricted to the illustrated example architectures or configurations but can be implemented using a variety of alternative architectures and configurations. Additionally, as would be understood by persons of ordinary skill in the art, one or more features of one embodiment can be combined with one or more features of another embodiment described herein. Thus, the scope of the present disclosure should not be limited by any one of the above-described exemplary embodiments.

It is also understood that any reference to an element herein using a designation such as "first," "second," and so forth does not generally limit the quantity or order of those elements. Rather, these designations can be used herein as a convenient means of distinguishing between two or more elements or instances of an element. Thus, a reference to first and second elements does not mean that only two elements can be employed, or that the first element must precede the second element in some manner. Additionally, a person having ordinary skill in the art would understand that information and signals can be represented using any one of a variety of different technologies and techniques. For example, data, instructions, commands, information, signals, bits and symbols, for example, which may be referenced in the above description can be represented by voltages, currents, electromagnetic waves, magnetic fields or particles, optical fields or particles, or any combination thereof.

A skilled person would further appreciate that any one of the various illustrative logical blocks, units, processors, means, circuits, methods and functions described in connection with the aspects disclosed herein can be implemented by electronic hardware (e.g., a digital implementation, an analog implementation, or a combination of the two), firmware, various forms of program or design code incorporating instructions (which can be referred to herein, for convenience, as "software" or a "software unit"), or any combination of these techniques.

To clearly illustrate this interchangeability of hardware, firmware and software, various illustrative components, blocks, units, circuits, and steps have been described above generally in terms of their functionality. Whether such functionality is implemented as hardware, firmware or software, or a combination of these techniques, depends upon the particular application and design constraints imposed on the overall system. Skilled artisans can implement the described functionality in various ways for each particular application, but such implementation decisions do not cause a departure from the scope of the present disclosure. In accordance with various embodiments, a processor, device, component, circuit, structure, machine, unit, etc. can be configured to perform one or more of the functions described herein. The term "configured to" or "configured for" as used herein with respect to a specified operation or function refers to a processor, device, component, circuit, structure, machine, unit, etc. that is physically constructed, programmed and/or arranged to perform the specified operation or function.

Furthermore, a skilled person would understand that various illustrative logical blocks, units, devices, components and circuits described herein can be implemented within or performed by an integrated circuit (IC) that can include a general purpose processor, a digital signal processor (DSP), an application specific integrated circuit (ASIC), a field programmable gate array (FPGA) or other programmable logic device, or any combination thereof. The logical blocks, units, and circuits can further include antennas and/or transceivers to communicate with various components within the network or within the device. A general purpose processor can be a microprocessor, but in the alternative, the processor can be any conventional processor, controller, or state machine. A processor can also be implemented as a combination of computing devices, e.g., a combination of a DSP and a microprocessor, a plurality of microprocessors, one or more microprocessors in conjunction with a DSP core, or any other suitable configuration to perform the functions described herein. If implemented in software, the functions can be stored as one or more instructions or code on a computer-readable medium. Thus, the steps of a method or algorithm disclosed herein can be implemented as software stored on a computer-readable medium.

Computer-readable media includes both computer storage media and communication media including any medium that can be enabled to transfer a computer program or code from one place to another. A storage media can be any available media that can be accessed by a computer. By way of example, and not limitation, such computer-readable media can include RAM, ROM, EEPROM, CD-ROM or other optical disk storage, magnetic disk storage or other magnetic storage devices, or any other medium that can be used to store desired program code in the form of instructions or data structures and that can be accessed by a computer.

In this document, the term "unit" as used herein, refers to software, firmware, hardware, and any combination of these elements for performing the associated functions described herein. Additionally, for purpose of discussion, the various units are described as discrete units; however, as would be apparent to one of ordinary skill in the art, two or more units may be combined to form a single unit that performs the associated functions according to embodiments of the present disclosure.

Additionally, memory or other storage, as well as communication components, may be employed in embodiments of the present disclosure. It will be appreciated that, for clarity purposes, the above description has described embodiments of the present disclosure with reference to different functional units and processors. However, it will be apparent that any suitable distribution of functionality between different functional units, processing logic elements or domains may be used without detracting from the present disclosure. For example, functionality illustrated to be performed by separate processing logic elements, or controllers, may be performed by the same processing logic element, or controller. Hence, references to specific functional units are only references to a suitable means for providing the described functionality, rather than indicative of a strict logical or physical structure or organization.

Various modifications to the implementations described in this disclosure will be readily apparent to those skilled in the art, and the general principles defined herein can be applied to other implementations without departing from the scope of this disclosure. Thus, the disclosure is not intended to be limited to the implementations shown herein, but is to be accorded the widest scope consistent with the novel features and principles disclosed herein, as recited in the claims below.

## Claims

1. A tracking marker (100) comprising:
a fiducial section (200) for providing a reference scale in at least two dimensions and an orientation reference; and
at least one color swatch (301, 302, 303, 304) with a plurality of color swatch elements for providing a color reference,
wherein the tracking marker (100) is preferably configured to be attached to an object in a 3D space,
**characterized in that**:
the fiducial section (200) is a cut-out in a central portion of the tracking marker (100), and
the at least one color swatch (301, 302, 303, 304) is arranged to surround the fiducial section (200);
wherein the tracking marker (100) has a square shape with a side length A,
wherein the tracking marker area is divided into n by m rectangular area elements with side lengths of A/n in a first direction and A/m in a second direction, with n and m being integer numbers, with n being preferably 6 and m being preferably 5,
wherein the tracking marker (100) comprises four color swatches (301, 302, 303, 304) and each color swatch (301, 302, 303, 304) comprises a same number of color swatch elements each corresponding to one rectangular area element, and
wherein the fiducial section (200) is a cut-out of the same number of rectangular area elements in the central portion of the tracking marker (100).

2. The tracking marker (100) according to claim 1,
wherein the at least one color swatch (301, 302, 303, 304) comprises at least one of: a set of natural colors, a set of primary and secondary colors, a set of grayscale colors, and a set of miscellaneous colors, wherein preferably each color swatch (301, 302, 303, 304) comprises six or more colors swatch elements.

3. The tracking marker (100) according to claim 1 or 2,
wherein the tracking marker (100) is a two-dimensional tracking marker (100), and the fiducial section (200) provides a reference scale in each of the two dimensions of the tracking marker (100).

4. The tracking marker (100) according to claim 1
wherein the rectangular area elements are arranged in m=6 columns in the first direction and n=5 rows in the second direction,
wherein a first color swatch (301) is arranged on the rectangular area elements of the first row,
wherein a second color swatch (302) is arranged on the rectangular area elements of the fifth row;
wherein a third color swatch (303) is arranged on the rectangular area elements of the second, third, and fourth row in the first and second column;
wherein a fourth color swatch (304) is arranged on the fourth to sixth rectangular area elements of the second row, on the sixth element of the third row, and the fifth and sixth rectangular area element of the fourth row, and
wherein the remaining rectangular area elements are forming the cut-out for the fiducial section (200).

5. A mobile device for scanning a 3D space provided with a plurality of tracking markers (100) according to any one of claims 1 to 4,
the mobile device comprising:
a camera configured to acquire a plurality of digital images of at least a part of the 3D space; and
a depth sensor configured to acquire corresponding depth data of the 3D space, and
wherein the mobile device comprises or is connected to a post processing unit configured to
correct the colors and/or white balance of the acquired images based on the at least one color swatch (301, 302, 303, 304) of the tracking markers; and
perform texture mapping based on the acquired images and based on position tracking data based on the fiducial section (200) of the tracking markers.

6. A post processing unit for use with a mobile device according to claim 5 for scanning a 3D space provided with a plurality of tracking markers (100) according to any one of claims 1 to 4,
wherein the post processing unit is configured to
correct the colors and/or white balance of the acquired images based on the at least one color swatch (301, 302, 303, 304) of the tracking markers; and
perform texture mapping based on the acquired images and based on position tracking data based on the fiducial section (200) of the tracking markers.

7. A system for scanning a 3D space comprising:
a plurality of tracking markers (100) according to any one of claims 1 to 4 arranged in the 3D space;
a mobile device according to claim 5; and
a post processing unit according to claim 6.

8. A method for scanning a 3D space provided with a plurality of tracking markers (100) according to any one of claims 1 to 4,
the method comprising:
acquiring with a camera of a mobile device a plurality of images of at least a part of the 3D space;
acquiring corresponding depth data of the 3D space with a depth sensor of the mobile device; and
performing a post processing comprising
correcting the colors and/or white balance of the acquired images based on the at least one color swatch (301, 302, 303, 304) of the tracking markers; and
performing texture mapping based on the acquired images and based on position tracking data based on the fiducial section (200) of the tracking markers.

## Patentansprüche

1. Trackingmarker (100), umfassend:
einen Fiducial-Abschnitt (200) zur Bereitstellung eines Referenzmaßstabs in zumindest zwei Dimensionen sowie einer Orientierungsreferenz; und
zumindest ein Farbfeld (301, 302, 303, 304) mit einer Vielzahl von Farbfeldelementen zur Bereitstellung einer Farbreferenz,
wobei der Trackingmarker (100) bevorzugt dafür ausgelegt ist, an einem Objekt in einem 3D-Raum befestigt zu werden,
**dadurch gekennzeichnet, dass**:
der Fiducial-Abschnitt (200) ein Ausschnitt in einem zentralen Bereich des Trackingmarkers (100) ist, und
das zumindest eine Farbfeld (301, 302, 303, 304) derart angeordnet ist, dass es den Fiducial-Abschnitt (200) umgibt;
wobei der Trackingmarker (100) eine quadratische Form mit einer Seitenlänge A aufweist,
wobei die Fläche des Trackingmarkers in n × m rechteckige Flächenelemente mit Seitenlängen von A/n in einer ersten Richtung und A/m in einer zweiten Richtung unterteilt ist, wobei n und m ganzzahlige Zahlen sind, wobei n bevorzugt 6 und m bevorzugt 5 ist,
wobei der Trackingmarker (100) vier Farbfelder (301, 302, 303, 304) umfasst und jedes Farbfeld (301, 302, 303, 304) die gleiche Anzahl von Farbfeldelementen aufweist, wobei jedes Farbfeldelement jeweils einem rechteckigen Flächenelement entspricht, und
wobei der Fiducial-Abschnitt (200) ein Ausschnitt derselben Anzahl von rechteckigen Flächenelementen im zentralen Bereich des Trackingmarkers (100) ist.

2. Trackingmarker (100) nach Anspruch 1,
wobei das zumindest eine Farbfeld (301, 302, 303, 304) zumindest eines der folgenden umfasst: einen Satz natürlicher Farben, einen Satz primärer und sekundärer Farben, einen Satz von Graustufenfarben und einen Satz sonstiger Farben, wobei bevorzugt jedes Farbfeld (301, 302, 303, 304) sechs oder mehr Farbfeldelemente umfasst.

3. Trackingmarker (100) nach Anspruch 1 oder 2,
wobei der Trackingmarker (100) ein zweidimensionaler Trackingmarker (100) ist und der Fiducial-Abschnitt (200) einen Referenzmaßstab in jeder der beiden Dimensionen des Trackingmarkers (100) bereitstellt.

4. Trackingmarker (100) nach Anspruch 1,
wobei die rechteckigen Flächenelemente in m = 6 Spalten in der ersten Richtung und n = 5 Reihen in der zweiten Richtung angeordnet sind,
wobei ein erstes Farbfeld (301) auf den rechteckigen Flächenelementen der ersten Reihe angeordnet ist,
wobei ein zweites Farbfeld (302) auf den rechteckigen Flächenelementen der fünften Reihe angeordnet ist;
wobei ein drittes Farbfeld (303) auf den rechteckigen Flächenelementen der zweiten, dritten und vierten Reihe in der ersten und zweiten Spalte angeordnet ist;
wobei ein viertes Farbfeld (304) auf den vierten bis sechsten rechteckigen Flächenelementen der zweiten Reihe, auf dem sechsten Element der dritten Reihe sowie auf dem fünften und sechsten rechteckigen Flächenelement der vierten Reihe angeordnet ist, und wobei die verbleibenden rechteckigen Flächenelemente den Ausschnitt für den Fiducial-Abschnitt (200) bilden.

5. Mobiles Gerät zum Scannen eines 3D-Raums, der mit einer Vielzahl von Trackingmarkern (100) nach einem der Ansprüche 1 bis 4 versehen ist, wobei das mobile Gerät umfasst:
eine Kamera, die dazu ausgelegt ist, eine Vielzahl digitaler Bilder von zumindest einem Teil des 3D-Raums aufzunehmen; und
einen Tiefensensor, der dazu ausgelegt ist, entsprechende Tiefendaten des 3D-Raums zu erfassen, und
wobei das mobile Gerät eine Nachverarbeitungseinheit umfasst oder mit dieser verbunden ist, die dazu ausgelegt ist,
die Farben und/oder den Weißabgleich der aufgenommenen Bilder auf Grundlage des zumindest einen Farbfelds (301, 302, 303, 304) der Trackingmarker zu korrigieren; und
Texturabbildung auf Grundlage der aufgenommenen Bilder sowie auf Grundlage von Positionsverfolgungsdaten durchzuführen, die auf dem Fiducial-Abschnitt (200) der Trackingmarker basieren.

6. Nachverarbeitungseinheit zur Verwendung mit einem mobilen Gerät nach Anspruch 5 zum Scannen eines 3D-Raums, der mit einer Vielzahl von Trackingmarkern (100) nach einem der Ansprüche 1 bis 4 versehen ist,
wobei die Nachverarbeitungseinheit dazu ausgelegt ist,
die Farben und/oder den Weißabgleich der aufgenommenen Bilder auf Grundlage des zumindest einen Farbfelds (301, 302, 303, 304) der Trackingmarker zu korrigieren; und
Texturabbildung auf Grundlage der aufgenommenen Bilder sowie auf Grundlage von Positionsverfolgungsdaten durchzuführen, die auf dem Fiducial-Abschnitt (200) der Trackingmarker basieren.

7. System zum Scannen eines 3D-Raums, umfassend:
eine Vielzahl von Trackingmarkern (100) nach einem der Ansprüche 1 bis 4, die im 3D-Raum angeordnet sind;
ein mobiles Gerät nach Anspruch 5; und
eine Nachverarbeitungseinheit nach Anspruch 6.

8. Verfahren zum Scannen eines 3D-Raums, der mit einer Vielzahl von Trackingmarkern (100) nach einem der Ansprüche 1 bis 4 versehen ist,
wobei das Verfahren umfasst:
Aufnehmen einer Vielzahl von Bildern von zumindest einem Teil des 3D-Raums mit einer Kamera eines mobilen Geräts;
Erfassen entsprechender Tiefendaten des 3D-Raums mit einem Tiefensensor des mobilen Geräts; und
Durchführen einer Nachverarbeitung, umfassend
Korrigieren der Farben und/oder des Weißabgleichs der aufgenommenen Bilder auf Grundlage des zumindest einen Farbfelds (301, 302, 303, 304) der Trackingmarker; und
Durchführen einer Texturabbildung auf Grundlage der aufgenommenen Bilder sowie auf Grundlage von Positionsverfolgungsdaten, die auf dem Fiducial-Abschnitt (200) der Trackingmarker basieren.

## Revendications

1. Un marqueur de suivi (100), comprenant :
une section fiducielle (200) destinée à fournir une échelle de référence dans au moins deux dimensions ainsi qu'une référence d'orientation ; et
au moins une plage de couleurs (301, 302, 303, 304) comprenant une pluralité d'éléments de plage de couleurs destinés à fournir une référence de couleur,
le marqueur de suivi (100) étant de préférence configuré pour être fixé à un objet dans un espace 3D,
**caractérisé en ce que** :
la section fiducielle (200) est une découpe dans une partie centrale du marqueur de suivi (100), et
la ou les plages de couleurs (301, 302, 303, 304) sont disposées de manière à entourer la section fiducielle (200) ;
le marqueur de suivi (100) ayant une forme carrée avec une longueur de côté A,
la surface du marqueur de suivi étant divisée en n × m éléments de surface rectangulaires ayant des longueurs de côté de A/n dans une première direction et de A/m dans une seconde direction, n et m étant des nombres entiers, n étant de préférence égal à 6 et m étant de préférence égal à 5,
le marqueur de suivi (100) comprenant quatre plages de couleurs (301, 302, 303, 304) et chaque plage de couleurs (301, 302, 303, 304) comprenant un même nombre d'éléments de plage de couleurs, chacun correspondant à un élément de surface rectangulaire, et
la section fiducielle (200) étant une découpe d'un même nombre d'éléments de surface rectangulaires dans la partie centrale du marqueur de suivi (100).

2. Le marqueur de suivi (100) selon la revendication 1,
dans lequel la ou les plages de couleurs (301, 302, 303, 304) comprennent au moins l'un des ensembles suivants : un ensemble de couleurs naturelles, un ensemble de couleurs primaires et secondaires, un ensemble de niveaux de gris, et un ensemble de couleurs diverses, de préférence chaque plage de couleurs (301, 302, 303, 304) comprenant six éléments de plage de couleurs ou plus.

3. Le marqueur de suivi (100) selon la revendication 1 ou 2,
dans lequel le marqueur de suivi (100) est un marqueur de suivi bidimensionnel (100), et la section fiducielle (200) fournit une échelle de référence dans chacune des deux dimensions du marqueur de suivi (100).

4. Le marqueur de suivi (100) selon la revendication 1,
dans lequel les éléments de surface rectangulaires sont disposés en m = 6 colonnes dans la première direction et n = 5 rangées dans la seconde direction,
dans lequel une première plage de couleurs (301) est disposée sur les éléments de surface rectangulaires de la première rangée,
dans lequel une deuxième plage de couleurs (302) est disposée sur les éléments de surface rectangulaires de la cinquième rangée ;
dans lequel une troisième plage de couleurs (303) est disposée sur les éléments de surface rectangulaires des deuxième, troisième et quatrième rangées dans la première et la deuxième colonne ;
dans lequel une quatrième plage de couleurs (304) est disposée sur les quatrième à sixième éléments de surface rectangulaires de la deuxième rangée, sur le sixième élément de la troisième rangée, ainsi que sur les cinquième et sixième éléments de surface rectangulaires de la quatrième rangée, et dans lequel les éléments de surface rectangulaires restants forment la découpe de la section fiducielle (200).

5. Un dispositif mobile destiné à scanner un espace 3D pourvu d'une pluralité de marqueurs de suivi (100) selon l'une quelconque des revendications 1 à 4, le dispositif mobile comprenant :
une caméra configurée pour acquérir une pluralité d'images numériques d'au moins une partie de l'espace 3D ; et
un capteur de profondeur configuré pour acquérir des données de profondeur correspondantes de l'espace 3D, et
dans lequel le dispositif mobile comprend ou est relié à une unité de post-traitement configurée pour
corriger les couleurs et/ou la balance des blancs des images acquises sur la base de la ou des plages de couleurs (301, 302, 303, 304) des marqueurs de suivi ; et
effectuer un mappage de texture sur la base des images acquises et sur la base de données de suivi de position fondées sur la section fiducielle (200) des marqueurs de suivi.

6. Une unité de post-traitement destinée à être utilisée avec un dispositif mobile selon la revendication 5 pour scanner un espace 3D pourvu d'une pluralité de marqueurs de suivi (100) selon l'une quelconque des revendications 1 à 4,
dans laquelle l'unité de post-traitement est configurée pour
corriger les couleurs et/ou la balance des blancs des images acquises sur la base de la ou des plages de couleurs (301, 302, 303, 304) des marqueurs de suivi ; et
effectuer un mappage de texture sur la base des images acquises et sur la base de données de suivi de position fondées sur la section fiducielle (200) des marqueurs de suivi.

7. Un système destiné à scanner un espace 3D, comprenant :
une pluralité de marqueurs de suivi (100) selon l'une quelconque des revendications 1 à 4 disposés dans l'espace 3D ;
un dispositif mobile selon la revendication 5 ; et
une unité de post-traitement selon la revendication 6.

8. Un procédé de scan d'un espace 3D pourvu d'une pluralité de marqueurs de suivi (100) selon l'une quelconque des revendications 1 à 4,
le procédé comprenant :
l'acquisition, au moyen d'une caméra d'un dispositif mobile, d'une pluralité d'images d'au moins une partie de l'espace 3D ;
l'acquisition de données de profondeur correspondantes de l'espace 3D au moyen d'un capteur de profondeur du dispositif mobile ; et
la réalisation d'un post-traitement comprenant
la correction des couleurs et/ou de la balance des blancs des images acquises sur la base de la ou des plages de couleurs (301, 302, 303, 304) des marqueurs de suivi ; et
la réalisation d'un mappage de texture sur la base des images acquises et sur la base de données de suivi de position fondées sur la section fiducielle (200) des marqueurs de suivi.
